# EUROPÄISCHE PATENTSCHRIFT

(11) **EP 0 996 438 B1**
(45) Veröffentlichungstag und Bekanntmachung des Hinweises auf die Patenterteilung: **24.08.2005**
(21) Anmeldenummer: 98919216.6
(22) Anmeldetag: 03.04.1998
(51) Int. Cl.: A61K 31/20, A61P 17/00, A61P 17/04, A61P 17/06, A61P 17/08

(54) **VERWENDUNG VON ESTERN DER CIS-6-HEXADECENSÄURE ZUR BEHANDLUNG VON PSORIASIS, ALLERGIEN UND AUTOIMMUNERKRANKUNGEN SOWIE BEI TROCKENER UND EMPFINDLICHER HAUT**
USE OF CIS-6-HEXADECENOIC ACID ESTERS FOR TREATING PSORIASIS, ALLERGIES, AUTO-IMMUNE DISORDERS AND DRY, SENSITIVE SKIN
UTILISATION D'ESTER D'ACIDE CIS-6-HEXADECENE POUR TRAITER LE PSORIASIS, DES ALLERGIES ET DES AFFECTIONS AUTO-IMMUNES, AINSI QU'EN CAS DE PEAU SECHE ET SENSIBLE

(30) Priorität: 11.06.1997 DE 19724622
(43) Veröffentlichungstag der Anmeldung: 03.05.2000
(73) Patentinhaber: Cognis IP Management GmbH, 40589 Düsseldorf (DE)
(72) Erfinder: HOPPE, Udo, D-24598 Heidmühlen (DE); JACOB, Jürgen, D-22397 Hamburg (DE)
(74) Vertreter: Fabry, Bernd
(86) Internationale Anmeldenummer: PCT/EP1998/001949
(87) Internationale Veröffentlichungsnummer: WO 1998/056371

(56) Entgegenhaltungen:
- WO-A-93/05752
- WO-A-94/21247
- US-A- 4 036 991
- DATABASE WPI Week 7519 Derwent Publications Ltd., London, GB; AN 75-31350W XP002072205 & JP 49 093 518 A (MIMATSU KAKO KK) , 5.September 1975

## Beschreibung

Gegenstand der Erfindung sind neue Verwendungen von Estern der cis-6-Hexadecensäure-(1). Sie wird auch cis-Hexadec-6-ensäure oder Delta-6-Hexadecensäure genannt.

Gesättigte und ungesättigte Fettsäuren sind Bestandteile der Zellmembran von Zellen. Die Konzentration von ungesättigten Fettsäuren spielt eine Rolle bei den Barriereeigenschaften der Haut und bei der Reaktivität von Zellen in entzündlichen Prozessen.

So ist bereits bekannt, cis-9-Heptadecensäure zur Behandlung von Psoriasis, Allergien und Autoimmunerkrankungen zu verwenden (DE-A-4309512).

Cis-6-Hexadecensäure wurde schon zum Weichmachen der Haut in kosmetischen und dermatologischen Zubereitungen vorgeschlagen (US-Patent 4,036,991). In der Literatur wurde auch schon beschrieben, daß cis-Hexadecensäure Bestandteil des menschlichen Hautfettes und der Psoriasisschuppen ist (Jacob et al., Z: Klin. Chem. Klin. Biochem., 11. Jg., (1973), 297-.300). Eine Kombination von Neutrallipiden mit cis-6-Hexadecensäure kann zur Behandlung von trockener Haut verwendet werden (DE-A-4131940).

Überraschenderweise wurde gefunden, daß Ester der cis-6-Hexadecensäure-(1) bei Psoriasis, Allergien und Autoimmunerkrankungen gegen Juckreiz sowie bei trockener Haut und bei empfindlicher Haut wirksam ist.

Gegenstand der Erfindung ist die Verwendung von Estern der cis-6-Hexadecensäure als Wirkstoff zur Prophylaxe und Behandlung von Psoriasis, Allergien und Autoimmunerkrankungen gegen Juckreiz sowie bei trockener Haut und bei empfindlicher Haut.

Die topische Applikation wird bevorzugt.

Gut geeignete Derivate der cis-6-Hexadecensäure, sind auch Naturstoffe, die diese Ester enthalten.

Bevorzugte Ester sind die Mono-, Di- oder Tri-Glyceride der cis-Hexadecensäure, gemischte Glyceride, die mindestens eine weitere Carbonsäure oder Fettsäure enthalten, die Mono- oder Di-cis-6-Hexadecensäureester mit Ethylenglykol und/oder Propylen-Glykol, wobei jeweils ein oder mehrere Mole Glykol pro Mol oder zwei Mol cis-Hexadecensäure enthalten sein können und cis-6-Hexadecensäureester mit geradkettigen oder verzweigten mono-Alkoholen mit z.B. 1 bis 22 Kohlenstoffatomen, insbesondere 12 bis 20 Kohlenstoffatomen im Alkylrest.

Weitere Carbonsäuren oder Fettsäuren der gemischten Triglyceride können z.B. 1 bis 22, vorzugsweise 12 bis 22 Kohlenstoffatome enthalten und gesättigt oder ungesätttigt sein und dann z.B. 1 bis 5, vorzugsweise 1 bis 3 Doppelbindungen besitzen.

Geeignete Mono- oder Diester mit Ethylenglykol und/oder Propylenglykol können z.B. 1 bis 50, vorzugsweise 5 bis 40, insbesondere 10 bis 30 Mol Glykol pro Mol des jeweiligen Esters enthalten.

Cis-6-Hexadecensäure ist Hauptbestandteil (85 Gew.-% als Triglycerid) des aus dem Saatgut gewonnenen Öles der Thunbergia alata, "Schwarzäugige Susanne" (G.F. Spencer et al., Lipids, Vol. 6, No. 10 und US-Patent 4,036,991).

Erfindungsgemäß können bevorzugt die natürlichen Triglyceride des Saatgutöles der Thunbergia alata mit cis-6-Hexadecensäure verwendet werden.

Erfindungsgemäß kann auch vorteilhaft als Naturstoff das Saatgutöl der Thunbergia alata verwendet werden. Es ist also z.B. nicht erforderlich, die Ester der cis-6-Hexadecensäure zu isolieren. Es ist auch möglich, die erfindungsgemäßen Wirkstoffe und insbesondere das Saatgutöl der Thunbergia alata in reiner Form ohne weitere Zusatzstoffe anzuwenden.

Aus dem Saatgut läßt sich das Öl in einfacher Weise, z.B. wie in der angesehenen Literatur beschrieben, erhalten. Aus dem Öl können dann nach bekannten Verfahren, beispielsweise durch die Esterspaltung, die cis-6-Hexadecensäure und daraus wiederum nach bekannten Verfahren die erfindungsgemäßen Derivate erhalten werden.

Mit der Bezeichnung Psoriasis ist diese Hauterkrankung in allen Erscheinungsformen gemeint.

Allergien sind insbesondere die Atopie und Kontaktallergien. Die Atopie manifestiert sich z.B. als allergische Konjunctivitis, allergische Rhinitis, allergisches Asthma oder insbesondere Neurodermitis.

Autoimmunerkrankungen sind insbesondere die Erkrankungen des rheumatischen Formenkreises.

Überraschenderweise sind die erfindungsgemäßen Wirkstoffe gegenüber so unterschiedlichen Krankheiten wie Psoriasis, Allergien und Autoimmunerkrankungen wirksam, sowie bei trockener Haut und bei empfindlicher Haut.

Die erfindungsgemäßen Wirkstoffe zeichnen sich durch eine starke antiflammatorische bzw. antientzündliche Wirkung aus.

Zur Prophylaxe werden die Wirkstoffe verabreicht, um Manifestationen der Krankheiten in der Häufigkeit und Stärke zu mindern. Die Behandlung im manifesten Stadium führt zu dessen Verkürzung und zur Milderung der Symptome.

Auch bei trockener und empfindlicher Haut können die Wirkstoffe prophylaktisch und zur Behandlung der Störungen angewendet werden.

Bei der trockenen Haut handelt es sich einerseits um Haut, der ein ausreichender oder normaler Feuchtigkeitsgehalt fehlt, andererseits aber auch um Haut, die in der Epidermis und Dermis unter Strukturschäden und Funktionsstörungen leidet, z.B. neben Trockenheit unter Rissigkeit und Ausbildung von Trockenheitsfältchen, Juckreiz (Pruritus) und verminderter Rückfettung durch Talgdrüsen (beispielsweise nach dem Waschen). Zu dem Begriff "trockene Haut" zählt auch die "senile Xerosis".

Die erfindungsgemäßen Wirkstoffe können auch bei empfindlicher Haut angewendet werden, insbesondere gegen neurosensorische Pänomene, z.B. "stinging".

Die Epidermis ist reich mit Nerven und Nervenendapparaten wie Vater-Pacini-Lamellenkörpersn, Merkel-Zell-Neuritenkomplexen und freien Nervenendigungen für Schmerz-, Kälte-, Wärmeempfindung und Juckreiz ausgestattet.

Bei Menschen mit sensibler, empfindlicher oder verletzlicher Haut kann daher ein "stinging" (englisch "sting" verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "stingings" bei sensibler Haut sind Rötung, Spannen und Brennen der Haut sowie Juckreiz.

Als weiteres neurosensorisches Phänomen ist der Juckreiz bei atopischer Haut anzusehen, sowie Juckreiz bei Hauterkrankungen.

Erfindungsgemäß gelingt es daher, Wirkstoffe und Zubereitungen mit solchen Wirkstoffen zur Verfügung zu stellen, welche insbesondere neurosensorische Phänomene verhindern oder nach dem Auftreten lindern oder schnell zum Abklingen bringen, also zur Prophylase und/oder Behandlung geeignet sind.

"Stinging"-Phänomene können als kosmetisch zu behandelnde Störungen angesehen werden. Starker Juckreiz dagegen, insbesondere bei Atopie, insbesondere Neurodermitis, auftretendes starkes Hautjucken, kann auch als schwenrviegendere dermatologische Störung bezeichnet werden.

Die erfindungsgemäßen Wirkstoffe können insbesondere auch an der oberflächlich gesund erscheinenden Haut, z.B. bei Psoriasis und Atopie, also auch neben den erkrankten Hautarealen angewendet werden und insbesondere auch hier bei trockener und empfindlicher Haut.
Als Salze bevorzugt werden wasserlösliche Salze der cis-9-Heptadecensäure, insbesondere die Alkalimetallsalze, z.B. das Natriumsalz oder das Kaliumsalz, und auch das Ammoniumsalz. Geeignet sind auch das Kalzium-, Magnesium-und Aluminiumsalz sowie die Salze organischer Basen, z.B. Aminen wie Äthanolamin, Äthylendiamin und Morpholin.

Gemäß der Erfindung werden auch pharmazeutische Präparate, Mittel oder Zusammensetzungen geschaffen, die die erfindungsgemäße Verbindung oder deren pharmazeutisch verträgliches Salz zusammen mit einem pharmazeutisch verträglichen Verdünnungsmittel oder Träger enthalten.

Die Verbindungen der vorliegenden Erfindung können beim Menschen oral oder parenteral z.B. in einer Dosierung von 0,05 bis 500 mg, vorzugsweise 0,5 bis 50 mg, besonders bevorzugt 0,1 bis 10 mg pro Tag angewendet werden, insbesondere auch in unterteilten Dosen, zum Beispiel zweimal bis viermal täglich.

Die erfindungsgemäßen Wirkstoffe lassen sich auch problemlos in übliche pharmazeutische, insbesondere dermatologische, und kosmetische Grundlagen für bevorzugte topische Applikationen einarbeiten und man erhält damit die entsprechenden pharmazeutischen, insbesondere dermatologischen, und kosmetischen topischen Zubereitungen oder Mittel. Bevorzugt werden sie in Mengen von 0,001 bis 10 Gew.-%, insbesondere in Mengen von 0,01 bis 1 Gew.-%, jeweils bezogen auf das gesamte Gewicht des topischen Mittels, eingesetzt. Auch Mengen von über 0,5 Gew.-%, z.B. 0,51 Gew.-% bis 10 Gew.-% werden bevorzugt, sowie auch Mengen im Bereich von 0,001 bis 0,05 bzw. 0,049 Gew.-% . Die Zubereitungen können mehrmals täglich in üblicher Weise angewendet werden.

Gegenstand der Erfindung ist auch die Verwendung der erfindungsgemäßen Wirkstoffe zur Herstellung von pharmazeutischen Mitteln, insbesondere topischen pharmazeutischen und kosmetischen Mitteln zur Prophylaxe und Behandlung von Psoriasis, Allergien und Autoimmunerkrankungen gegen Juckreiz sowie bei trockener Haut und bei empfindlicher Haut.

Ebenfalls Gegenstand der Erfindung ist auch die Verwendung von pharmazeutischen Mitteln und topischen pharmazeutischen und kosmetischen Zubereitungen mit einem Gehalt an Estern der cis-6-Hexadecensäure zur Prophylaxe und Behandlung von Psoriasis, Allergien und Autoimmunerkrankungen gegen Juckreiz sowie bei trockener Haut und bei empfindlicher Haut.

Die Ester der cis-6-Hexadecensäure zeigten im Makrophagen-Differenzierungstest eine antientzündliche Makrophagen stimulierende Potenz. Dies ist bei der Prophylaxe von Prozessen, wie z.B. Psoriasis oder Atopie oder Allergien oder Autoimmunerkrankungen von Bedeutung.

Gegenstand der Erfindung ist auch die Verwendung der erfindungsgemäßen Wirkstoffe als antibakterielle Wirkstoffe, z.B. in den genannten Zubereitungen, insbesondere in topischen Zubereitungen, z.B. in den genannten Mengen.

Sie werden bevorzugt gegen grampositive Bakterien, insbesondere gegen Micrococcus luteus verwendet.

Die Wirkstoffe gemäß der Erfindung können mit üblichen pharmazeutisch verträglichen Verdünnungsmitteln oder Trägem und gegebenenfalls mit anderen Hilfsmitteln vermischt und beispielsweise oral oder parenteral verabreicht werden. Sie können vorzugsweise oral in Form von Granulaten, Kapseln, Pillen, Tabletten, Filmtabletten, Dragees, Sirupen, Emulsionen, Suspensionen, Dispersionen, Aerosolen und Lösungen sowie Flüssigkeiten, oder aber auch als Zäpfchen, Vaginalkugeln oder parenteral z.B. in Form von Lösungen, Emulsionen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Prophylenglycol, Ether des Tetrahydrofurfurylalkohols und Wasser.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calziumcarbonat und Dicalziumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert.

Die pharmazeutischen Präparate können den Wirkstoff in einer Menge von 0,1 bis 90 Gewichtsprozent, insbesondere 1-90 Gew.-% enthalten. Kapseln werden besonders bevorzugt. Einzeldosen enthalten die Wirkstoffe vorzugsweise in einer Menge von 1 bis 10 mg.

Soweit Salze in Wasser schwer löslich sind, können sie in der Form von Aufschlämmungen verabreicht werden. Eine besonders gute Löslichkeit in Wasser besitzen die Natrium- und die Kaliumsalze der cis-9-Heptadecensäure. Beispielsweise werden Salze vorzugsweise in der Form einer wäßrigen Lösung, wie physiologische Kochsalzlösung, intravenös oder intramuskulär gespritzt. Die Ampullen enthalten z.B. 2,5 mg des fettsauren Salzes je 5 ml Lösung. Man kann auch Ampullen mit z.B. 45 mg fettsaurem Salz je Milliliter Lösung herstellen.

Die besonders bevorzugten erfindungsgemäßen topischen Mittel können als flüssige, pastöse oder feste Zubereitungen formuliert werden, beispielsweise als wäßrige oder alkoholische Lösungen, wäßrige Suspensionen, Emulsionen, beispielsweise W/O- oder O/W-Emulsionen, Salben, Gele, Lotionen, Cremes, Öle, Pulver oder Stifte. In Abhängigkeit von der gewünschten Formulierung können die Wirkstoffe in pharmazeutische und kosmetische Grundlagen für topische Applikationen eingearbeitet werden, die als weitere Komponenten beispielsweise Ölkomponenten, Fett und Wachse, Emulgatoren, anionische, kationische, ampholytische, zwitterionische und/oder nichtionogene Tenside, niedere ein- und mehrwertige Alkohole, Wasser, Konservierungsmittel, Puffersubstanzen, Verdickungsmittel, Duftstoffe, Farbstoffe und Trübungsmittel enthalten. Bevorzugt werden Emulsionen, z.B. W/O-Emulsionen oder Salben verwendet.

Weiterhin ist es erfindungsgemäß bevorzugt, den Wirkstoffen und den pharmazeutischen und topischen Zubereitungen Antioxidantien zuzufügen. Besonders bevorzugt ist hierbei die Verwendungen natürlicher oder naturidentischer Verbindungen wie beispielsweise Tocopherolen. Die genannten Antioxidantien sind in den erfindungsgemäßen Mitteln z.B. in Mengen von 0,01 - 5 Gew.-%, insbesondere von 0,5 - 2 Gew.-%, bezogen auf das gesamte Mittel, enthalten.

Im Rahmen der vorliegenden Anmeldung sind, soweit nicht anders angegeben, Mengen und Prozentangaben auf das Gewicht und die Gesamtzusammensetzung oder Zubereitung bezogen.

### Beispiel 1

| | |
|---|---|
| Polyoxyethylen(20)sorbitan-monostearat (Polysorbat 60) | 5 |
| Cetyl/Stearylalkohol (Cetostearylalkohol) | 10 |
| Glycerol 85% | 10 |
| Weißes Vaselin | 25 |
| a-D-Tocopherol | 1 |
| cis-6-Hexadecensäure-Triglycerid | 1 |
| gegebenenfalls Farbstoffe, Duftstoffe | |
| Wasser | ad 100 |

Die Herstellung erfolgt in an sich bekannter Weise. Die Fettphase und die Wasserphase werden separat durch Mischen der Bestandteile hergestellt, gegebenenfalls unter geringer Erwärmung. Dann werden die Phasen gemischt und emulgiert.

### Beispiel 2

| Creme: | |
|---|---|
| | Gewichtsteile |
| Polyoxyethylen(20)sorbitan- | |
| monostearat (Polysorbat 60) | 5 |
| Cetyl/Stearylalkohol (Cetostearylalkohol) | 10 |
| Glycerol 85% | 10 |
| Weißes Vaselin | 25 |
| a-D-Tocopherol | 1 |
| cis-6-Hexadecensäure-Triglyerid | 0,1 |
| gegebenenfalls Farbstoffe, Duftstoffe, | |
| Wasser | ad 100 |

Die Herstellung erfolgt in an sich bekannter Weise. Die Fettphase und die Wasserphase werden separat durch Mischen der Bestandteile hergestellt, gegebenenfalls unter geringer Erwärumung. Dann werden die Phasen gemischt und emulgiert.

### Beispiel 3

| Creme: | |
|---|---|
| | Gewichtsteile |
| Polyoxyethlyen(29)sorbitan- | |
| monostearat (Polysorbat 60) | 5 |
| Cetyl/Stearylalkohol (Cetostearylalkohol) | 10 |
| Glycerol 85% | 10 |
| Weißes Vaselin | 25 |
| a-D-Tocopherol | 1 |
| Saatgutöl der Thunbergia alata | 1 |
| gegebenenfalls Farbstoffe, Duftstoffe | |
| Wasser | ad 100 |

### Beispiel 4

| Creme: | |
|---|---|
| | Gewichtsteile |
| Polyoxyetylen(20)sorbitan- | |
| monostearat (Polysorbat60) | 5 |
| Cetyl/Stearylalkohol (Cetostearylalkohol) | 10 |
| Glycerol 85% | 10 |
| Weißes Vaselin | 25 |
| a-D-Tocopherol | 1 |
| Saatgutöl der Thunbergia alata | 0,1 |
| gegebenenfalls Farbstoffe, Duftstoffe | |
| Wasser | ad 100 |

Die Herstellung erfolgt in an sich bekannter Weise. Die Fettphase und die Wasserphase werden separat durch Mischen der Bestandteile hergestellt, gegebenenfalls unter geringer Erwärmung. Dann werden die Phasen gemischt und emulgiert.

## Patentansprüche

1. Verwendung von Estern der cis-6-Hexadecensäure zur Herstellung eines Mittels zur Prophylaxe und Behandlung von Psoriasis, Allergien und Autoimmunerkrankungen, gegen Juckreiz, sowie bei trockener und bei empfindlicher Haut, wobei auch Naturstoffe verwendet werden, die Ester der cis-6-Hexadecensäure enthalten.

2. Verwendung von Estern der cis-6-Hexadecensäure zur Herstellung eines Mittels mit antiinflammatorischer Wirkung, wobei auch Naturstoffe verwendet werden, die Ester der cis-6-Hexadecensäure enthalten.

3. Verwendung nach den Ansprüchen 1 und 2, **dadurch gekennzeichnet, dass** das Mittel eine pharmazeutische oder kosmetische topische Zubereitung ist.

4. Verwendung nach den Ansprüchen 1 bis 3, **dadurch gekennzeichnet, dass** als Ester der cis-6-Hexadecensäure Mono-, Di- und Triglyceride eingesetzt werden.

5. Verwendung nach den Ansprüchen 1 bis 3, **dadurch gekennzeichnet, dass** der Naturstoff das Saatgutöl der Pflanze Thunbergia alata ist.

6. Verwendung nach den Ansprüchen 1 bis 5, **dadurch gekennzeichnet, dass** zusätzlich ein Antioxidans verwendet wird.

## Claims

1. The use of esters of cis-6-hexadecenoic acid for the production of a preparation for the prophylaxis and treatment of psoriasis, allergies and autoimmune diseases, against itching and in cases of dry skin and sensitive skin, natural substances containing esters of cis-6-hexadecenoic acid also being used.

2. The use of esters of cis-6-hexadecenoic acid for the production of an anti-inflammatory preparation, natural substances containing esters of cis-6-hexadecenoic acid also being used.

3. The use claimed in claims 1 and 2, **characterized in that** the preparation is a pharmaceutical or cosmetic topical preparation.

4. The use claimed in claims 1 to 3, **characterized in that** mono-, di-and triglycerides are used as the esters of cis-6-hexadecenoic acid.

5. The use claimed in claims 1 to 3, **characterized in that** the natural substance is the seed oil of the plant Thunbergia alata.

6. The use claimed in claims 1 to 5, **characterized in that** an antioxidant is additionally used.

## Revendications

1. Utilisation d'esters de l'acide cis-6-hexadécénoïque pour la fabrication d'un produit pour la prophylaxie et le traitement du psoriasis, des allergies et des maladies auto-immunes, contre le prurit, ainsi que pour la peau sèche et la peau sensible, selon laquelle on utilise également des produits naturels qui contiennent des esters de l'acide cis-6-hexadécénoïque.

2. Utilisation d'esters de l'acide cis-6-hexadécénoïque pour la fabrication d'un produit à action anti-inflammatoire, selon laquelle on utilise également des produits naturels qui contiennent des esters de l'acide cis-6-hexadécénoïque.

3. Utilisation selon les revendications 1 et 2,
**caractérisée en ce que**
le produit est une préparation pharmaceutique ou cosmétique topique.

4. Utilisation selon les revendications 1 à 3,
**caractérisée en ce que**
comme esters de l'acide cis-6-hexadécénoïque, on utilise, des mono-, di-et triglycérides.

5. Utilisation selon les revendications 1 à 3,
**caractérisée en ce que**
le produit naturel est l'huile de semences de la plante Thunbergia alata.

6. Utilisation selon les revendications 1 à 5,
**caractérisée en ce qu'**
on utilise en outre un antioxydant.
